# EUROPÄISCHE PATENTSCHRIFT

(11) **EP 0 554 656 B1**
(45) Veröffentlichungstag und Bekanntmachung des Hinweises auf die Patenterteilung: **14.07.1999**
(21) Anmeldenummer: 93100039.2
(22) Anmeldetag: 05.01.1993
(51) Int. Cl.: G01N 33/564, G01N 33/536, G01N 33/543

(54) **Verfahren zur Bestimmung von Rheumafaktoren und Mittel zur Durchführung des Verfahrens**
Method and means for determination of rheumatoid factors
Méthode et moyens pour la détermination des facteurs rhumatoides

(30) Priorität: 01.02.1992 DE 4202924
(43) Veröffentlichungstag der Anmeldung: 11.08.1993
(73) Patentinhaber: Dade Behring Marburg GmbH, 35001 Marburg (DE)
(72) Erfinder: Toth, Tibor, W-3550 Marburg (DE)

(56) Entgegenhaltungen:
- EP-A- 0 019 741
- EP-A- 0 044 041
- EP-A- 0 087 728
- EP-A- 0 353 306
- US-A- 4 547 466
- DATABASE WPIL, Section Ch, Week 8845, Derwent Publications Ltd., London (GB); Class B04, AN 88-319016/
- DATABASE WPIL, Section Ch, Week 8514, Derwent Publications Ltd., London (GB); Class A96, AN 85-084828/

## Beschreibung

Die Erfindung betrifft ein immunchemisches Verfahren zum Nachweis und zur Bestimmung von Rheumafaktoren unter Verwendung von Immunkomplexen als spezifische Bindungspartner sowie die Herstellung von für diese Verfahren geeigneten Reagenzien.

Serologische Untersuchungsmethoden haben in der rheumatologischen Diagnostik breite Anwendung gefunden. Dabei werden Seren auf das Vorhandensein von Antikörpern untersucht, die der Körper im Fall dieser Erkrankungen gegen körpereigene Substanzen bildet; diese Antikörper werden daher auch als "Autoantikörper" bezeichnet.

In der Rheumadiagnostik haben sich vor allem zwei Gruppen von Autoantikörpern als wichtig erwiesen: Rheumafaktoren (RF) und anti-nukleäre Antikörper (ANA). Rheumafaktoren sind Antikörper gegen körpereigene Immunglobuline, also anti-Immunglobulin-Antikörper, anti-nukleäre Antikörper sind Autoantikörper gegen Strukturen körpereigener Zellkerne.
Rheumafaktoren kommen in allen Immunglobulinklassen vor, sie gehören meist zu den Immunglobulinklassen IgM, IgG und IgA.

Bekannte Testmethoden zum Nachweis von rheumatoiden Faktoren beruhen auf der Agglutination von beispielsweise Erythrozyten oder Latexpartikeln, die mit humanem oder tierischem IgG beschichtet sind. Diese Verfahren erlauben nur eine qualitative oder semiquantitative Bestimmung von Rheumafaktoren und die Ablesung der Ergebnisse dieser Agglutinations-Tests ist, bedingt durch die visuelle Auswertung, subjektiv.

Neben Agglutinationsmethoden (Latex-Agglutination, Waaler-Rose-Test), Radio- und Enzymimmunoassays, werden nephelometrische oder turbidimetrische Methoden zur Bestimmung von Rheumafaktoren herangezogen. Die Lichtstreuung und somit die Empfindlichkeit der nephelometrischen bzw. turbidimetrischen Methode ist von der "Partikelgröße" des Antigens bzw. des Antikörpers abhängig. Eine bekannte Methode besteht darin, ein humanes Immunglobulin an Latexpartikel, die selbst das Licht streuen, zu binden.

Radio- und Enzymimmunoassay erlauben neben einer quantitativen Auswertung auch eine Differenzierung der Rheumafaktoren-Klassen IgM, IgG und IgA. Sie sind jedoch sehr arbeitsintensiv und die Bestimmung dauert mehrere Stunden.

Wie aus den Arbeiten von J. Grange und Mitarbeitern in J. Immunol. Meth. 18 (1977) 365-375 hervorgeht, nimmt bei der Titration eines an Latex gebundenen Antikörpers mit Antigen zunächst einmal das Streulichtsignal ab, weil mit zunehmender Agglutination die Teilchenzahl abnimmt. Danach läßt ein weiterer Anstieg der Antigen-Konzentration die Aggregatgröße ansteigen, womit die Streulichtintensität zunimmt, bis bei weiterem Anstieg der Antigenmenge im Antigenüberschuß das Streulichtsignal wieder abnimmt. Für den quantitativen Test ist es wichtig, die Agglutinationsbedingungen des Latex so zu kontrollieren, daß bei einer Titration von Latex-gebundenen Antikörpern mit Antigen oder von Latex-gebundenem Antigen mit Antikörpern die Zunahme der Agglutination in weitem Bereich als Zunahme der Streulichtintensität gemessen werden kann.

Bei den partikelverstärkten Agglutinationstests für die Rheumafaktorenbestimmung muß zunächst eine Verdünnungsreihe der Seren gemacht werden. Nachteilig sind ein nicht eindeutiger Verlauf der Streulichtintensität bei der Titration, ein sehr schmaler Meßbereich, d. h. ein steiler Anstieg der Referenzkurve, sowie hohe Blindwerte des Latexreagenzes.

Es wurde u. a. vorgeschlagen (Deutsche Offenlegungsschrift DE 29 18 342), Latexpartikel zweier verschiedener Größenbereiche zu mischen, um den Meßbereich zu erweitern. Zusätzlich sind diese Latexpartikel verschiedener Größenbereiche jeweils mit mindestens einer von der anderen verschiedenen Menge von Antikörpern beladen. Nachteilig ist die Inkubationszeit von bis zu 1 Stunde sowie die Messung der Lichtstreuung nach weiteren 15 min am Laser Nephelometer. Es wurden in der letzten Zeit weitere z. T. automatisierbare Bestimmungsmethoden für Rheumafaktoren beschrieben, die jedoch auch nicht die Probleme, wie Verdünnungsechtheit, hohe Empfindlichkeit und große Meßbereiche zu lösen vermögen.

In US 4,547,466, im Abstract Nr. 85-084828 & JP-A-60036966 (Derwent Publications) und in EP-A-0 353 306 ist jeweils die Bestimmung von Rheumafaktoren mit einem Reagenz beschrieben, welches durch Bindung eines Antigens (z.B. Rinderserumalbumin) an Latexpartikel oder an eine Methacrylat-Mikrotitrationsplatte und einer darauffolgenden Reaktion der Antigenbeschichteten Festphase mit gegen das Antigen gerichteten Antikörpern hergestellt wird.

Es wurde nun überraschenderweise gefunden, daß diese Schwierigkeiten beseitigt werden können, wenn statt eines humanen Gammaglobulins ein Immunkomplex aus Antikörpern gegen human-IgG vom Schaf und human-IgG oder ein Immunkomplex aus Antikörpern gegen human-Gammaglobulin vom Kaninchen und human-Gammaglobulin verwendet werden. Die Immunkomplexe werden in wässriger Lösung hergestellt und nach bekannten Methoden an Latex-Partikel gebunden. Das erhaltene Latex-Reagenz ist verwendbar im manuellen Latex-Agglutinationstest oder in nephelometrischen bzw. turbidimetrischen Methoden nach kinetischen oder Endpunktmethoden.

Gegenstand der Erfindung ist demnach ein immunchemisches Verfahren zum Nachweis und zur Bestimmung von Rheumafaktoren (Analyt) mittels eines spezifischen Bindungspartners, wobei dieser ein Immunkomplex ist, welcher durch Mischen eines Antigens und eines dagegen gerichteten Antikörpers in wässriger Lösung hergestellt und anschließend an einer Festphase immobilisiert wird.

Bevorzugt ist dabei ein Verfahren, wobei der Immunkomplex aus Antiseren von immunisierten Tieren mit einem Antigen humanen, tierischen oder pflanzlichen Ursprungs hergestellt wurde, das nicht der Analyt ist.

Besonders bevorzugt ist ein Verfahren in dem das Verhältnis im Immunkomplex von Antikörper : Antigen = 1 : 0,05 bis 5 beträgt.

Bevorzugterweise wird ein Antiserum vom Schaf oder vom Kaninchen verwendet.

Bevorzugt ist ferner ein Verfahren wie oben beschrieben, wobei die Auswertung nach einer partikelverstärkten immunologischen Methode erfolgt.

Außerdem ist Gegenstand der Erfindung ein Verfahren zur Herstellung eines Reagenzes zur Verwendung in dem ohne beschriebenen Verfahren, wobei der Immunkomplex in einer wäßrigen Lösung in Gegenwart von bis zu 50 Volumen % eines zyklischen Amids hergestellt wird, bevorzugterweise in Gegenwart von gamma-Amino-Butyrolactam und anschließend ein einer Festphase immobilisiert wird.

Die Latexteilchen können adsorptiv oder durch kovalente Bindung von Proteinen beladen werden. Zur kovalenten Bindung können Latexderivate mit den freien Carboxylgruppen, Aminogruppen, Aldehydgruppen und Epoxygruppen sowie vicinalen Hydroxylgruppen herangezogen werden.

In vielen Fällen erwies sich die adsorptive Bindung von Proteinen an das Latex als am günstigsten.

Verfahren zur Beladung von Latexteilchen mit Proteinen und/oder Peptiden sind dem Fachmann bekannt.

Die zur Herstellung des erfindungsgemäßen Reagenzes verwendbaren Latices können beispielsweise durch Polymerisation olefinisch ungesättigter Monomere erhalten werden. Bevorzugt sind Styrol-Copolymerisate, wie Styrol-Butadien-Copolymerisate und Acrylnitril-Butadien-Styrol-Copolymerisate, Vinylacetat-Acrylat-Copolymerisate oder Vinylchlorid-Acrylat-Copolymerisate.

Polystyrol-Latex-Suspensionen oder Emulsionen geeigneter Teilchengröße (50 - 500 nm) können auch unter verschiedenen Handelsbezeichnungen von einer Anzahl von Herstellern erhalten werden. Geeignet sind mono- und polydisperse Latex-Suspensionen.

Die Immunkomplexe können nach bekannten Verfahren durch Vermischen des Antigens mit dem spezifischen Antikörper hergestellt werden. Geeignet sind spezifische Antiseren, Gammaglobulinfraktionen aus Antiseren, immunadsorptiv gereinigte Antikörper oder monoklonale Antikörper. Auch Seren und Gammaglobulinfraktionen aus Antiseren, die bereits durch die Immunisierung von Tieren Immunkomplexe enthalten, sind geeignet. Beispiele für Immunkomplexe sind human-IgG/Antiserum gegen human-IgG vom Schaf, human-Gammaglobulin/Antiserum gegen human-Gammaglobulin vom Kaninchen. Das Verhältnis von Antigen/Antikörper im Immunkomplex hängt vom Antikörpergehalt ab und beträgt 1 : 0,05 - 5, bevorzugterweise 0,05 : 2, ganz bevorzugterweise etwa 1 : 0,1.

Gegenstand der Erfindung ist also auch ein Verfahren für die Herstellung des in dem Verfahren verwendeten Reagenzes. Da bei der partikel-verstärkten Agglutinationsmethode meist ein Fällungsmittel wie Polyäthylenglycol (PEG) verwendet wird, dürfen die Komplexe nicht durch das Fällungsmittel ausgefällt werden. Außerdem müssen sie über einen längeren Zeitraum, daß heißt in der Regel mindestens 3 Monate, bevorzugterweise mindestens 12 Monate, lagerstabil sein.

Die Herstellung des Komplexes erfolgt in einer wäßrigen Lösung, vorzugsweise in Gegenwart eines polaren mit Wasser gut mischbaren Lösungsmittels wie Dimethylsulfoxid, Dimethylformamid. Bevorzugt ist die Herstellung der Komplexe in Gegenwart eines zyklischen Amids, besonders geeignet ist Pyrrolidon (gamma-Amino-Butyrolactam). Die so hergestellten Immunkomplexe sind in wäßrigen Lösungen mindestens 3, bevorzugterweise mindestens 12 Monate lagerstabil.

Die Beladung der Latex-Teilchen mit dem Immunkomplex kann nach einer dem Fachmann bekannten Methode durchgeführt werden. Es kann z. B. wie folgt beladen werden:

Ein aus einem Antiserum/Antigen hergestellter Immunkomplex wird mit einer Suspension der Latex-Teilchen mit einer Konzentration von etwa 50 bis 200, bevorzugterweise 100 g/l versetzt und 0,5 - 5 Stunden bei einer Temperatur zwischen 0 und +60 °C, bevorzugterweise +20 - +60 °C inkubiert.

Der nicht an Latex-Teilchen gebundene Anteil am Immunkomplex kann durch Zentrifugation und Resuspension des Feststoffes entfernt werden. Zum Gebrauch kann das Reagenz in einer Pufferlösung, vorzugsweise Glycin-NaCl-Puffer von pH 7 - 8,5 resuspendiert werden, die gegebenenfalls mit einem Protein, beispielsweise mit human-Albumin oder Rinder-Albumin, versetzt werden kann.

Nach einer anderen Methode für die Beladung der Latex-Teilchen wird der Immunkomplex in einem Glycin-NaCl-Puffer vorgelegt, ggf. mit human- oder Rinderserum-Albumin stabilisiert und mit einer 0,5 bis 2 %igen Polystyrol-Latex-Suspension bis zum Erreichen der gewünschten Empfindlichkeit versetzt. Das so hergestellte Reagenz kann bei +4 °C gelagert oder lyophilisiert werden.

Die zur Herstellung des Antikörpers verwendbaren Antiseren werden durch Immunisieren von Tieren, besonders Kaninchen, Schafen, Ziegen mit Proteinen humanen, tierischen oder pflanzlichen Ursprungs erhalten. Beispiele sind: anti-human-IgG-Serum vom Kaninchen, anti-human-Gammaglobulin vom Kaninchen, anti-Kaninchen-Gammaglobulin-Serum vom Schaf, anti-Human-IgG-Serum vom Schaf.

Besonders geeignet sind das anti-human-IgG-Serum vom Schaf und anti-human-Gammaglobulin-Serum vom Kaninchen. Die Immunisierung wurde nach bekannten Methoden durchgeführt. Immunisierungsdosis und Zeit ergeben sich aus der Immunogenität und dem Molekulargewicht des Proteins.

Als Antikörper im Sinne dieser Erfindung können auch monoklonale Antikörper eingesetzt werden.

Die folgenden Beispiele erläutern das erfindungsgemäße Verfahren:

### Beispiel 1

A) Herstellung des Immunkomplexes
   56 ml (16 g/l) Human-Gammaglobulin-Lösung in isotonischer Kochsalzlösung werden mit 25 ml Antiserum gegen Human-Gammaglobulin und 25 ml isotonischer Kochsalzlösung unter intensiver Rührung zusammengegeben. Die Lösung wurde danach 5 Std. bei +56 °C inkubiert und mit 5 ml Pyrrolidon versetzt.
B) Herstellung des Latex-Reagenzes
   Es wurden 6 ml Immunkomplex und 450 ml (10 g/l) Polystyrol-Latex eingesetzt. Für die nephelometrische Messung wurde diese Suspension auf 0,065 g/l Feststoff verdünnt und mit Ultraschall behandelt. Es wurde ein Standardserum mit 70 IU/ml Rheumafaktoren verwendet, die Standard-Reihe automatisch am Gerät 1:2,5 bis 160 verdünnt, d. h. es wurden RF-Konzentrationen von 28 IU, 14, 7, ... 0,45 IU/ml erhalten. Die zu bestimmenden Seren wurden in einer Phosphat-Kochsalz-Pufferlösung verdünnt. Zur Messung wurden 30 µl Patientenserumverdünnung und 40 µl des Latex-Immunkomplex-Reagenzes eingesetzt. Die Messung erfolgte nach 6 min Raumtemperatur an einem Nephelometer (BNA, Behringwerke AG, Marburg). Die Referenzkurve für die Messung des Standardserums wurde aufgezeichnet und daran wurden die Meßwerte der Patientensera ausgewertet.
   Seren mit bekannten RF-Konzentrationen wurden getestet. Der Meßbereich betrug bei einer 1:20-Serumverdünnung von 18 bis 560 IU/ml (Normalbereich: ≤ 20 IU RF/ml).
C) Latex-Immunkomplex-Reagenz wurde zur Messung von Rheumafaktoren in Patientenseren eingesetzt. Der RF-Standard enthielt 70 IU RF/ml. Der Standard wurde am Nephelometer mit Phosphat-Kochsalz-Puffer stufenweise verdünnt. Man erhielt so die Standardreihe mit abnehmenden RF-Konzentrationen.

Die zu bestimmenden Patientensera wurden in Phosphat-Kochsalz-Puffer verdünnt. Zur Messung wurden 30 µl Patientenserumverdünnung, beziehungsweise Standardserum-Verdünnung mit 40 µl des Latex-Immunkomplex-Reagenzes und 30 µl einer Reaktionspufferlösung, die 1,2 g/100 ml NaCl, 1,3 g/100 ml Na₂HPO₄, 0,2 g/100 ml Na₂PO₄ und 5,6 g Polyethylenglykol 6000 enthielt, 6 min bei Raumtemperatur inkubiert. Die Ergebnisse wurden dann an einem Nephelometer (Behringwerke AG, Marburg) gemessen.

Der Meßbereich betrug bei einer 1:20-Serumverdünnung 9 bis 560 IU/ml, bei einer 1:100-Serumverdünnung 45 bis 7000 IU/ml.

| Serum Nr. | RF IU/ml Serumverdünnung | |
|---|---|---|
| | 1:20 | 1:100 |
| 1 | 393 | 401 |
| 2 | 328 | 316 |
| 3 | 181 | 180 |
| 4 | < 9 | < 45 |
| 5 | 195 | 186 |
| Serum 4 war negativ. | | |

Die Tabelle zeigt die sehr gute Wiederfindung und Verdünnungsechtheit der Bestimmung der Rheumafaktoren nach der Erfindung.

### Beispiel 2

A) Beschichtung der Mikrotitrationsplatten
   Ein Immunkomplex (hergestellt nach 1 A) wurde in 0,05 Mol/g Natriumphosphatpuffer, pH 6,6 auf eine Konzentration von 12 µg/ml verdünnt. In jede Kavität einer Mikrotitrationsplatte (z.B. Fa. NUNC) wurden 135 µl der Verdünnung dispensiert.
   Die Platten wurden mit einer Folie abgedeckt und über Nacht bei Raumtemperatur inkubiert. Nach Inkubation wurden die Platten mit je 250 µl 0,1 Mol/l Tris-Citratpuffer, pH 4,9 pro Kavität zweimal gewaschen und an der Luft getrocknet.
B) Testdurchführung
   1) Alle Reagenzien und Proben wurden auf +18 bis +25°C erwärmt. Die Proben wurden 1:101 (10 µl + 1 ml) in einem proteinhaltigen Trispuffer, pH 8,2 verdünnt und gut gemischt.
   2) Je 100 µl der verdünnten Proben, Kontrollen und Standards wurden pro Kavität eingefüllt, die befüllte Testplatte wurde abgedeckt und 60 min bei +37°C (+ 1°C) inkubiert.
   3) Danach wurde die Testplatte 4mal mit jeweils 0,3 ml Tween-haltiger Phosphat-Pufferlösung gewaschen.
   4) Pro Kavität wurden anschließend 100 µl Anti-Human-IgM/POD-Konjugat eingefüllt, 60 min bei +37°C inkubiert und wie unter Punkt 3 gewaschen.
   5) In jede Kavität wurden 100 µl Chromogen-Puffer/Substrat-Lösung (Tetramethylbenzidin-Dihydrochlorid in einer wasserstoffperoxid-haltigen Acetat-Pufferlösung) eingefüllt, die Platte abgedeckt und 30 min bei +20 bis +25°C lichtgeschützt inkubiert. Nach Einfüllung von 100 µl Stopplösung (0,5 N Schwefelsäure) pro Kavität wurde die Platte bei 450 µm Meßwellenlänge und 650 µm Referenzwellenlänge photometriert. Die Auswertung des Testes erfolgte mit Hilfe einer Standardkurve (polygonale Interpolation) anhand der Extinktionswerte der Standards.

Folgende Tabelle zeigt eine Gegenüberstellung von RF-Werten, die mit Hilfe des Immukomplex-ELISA's und mit einem quantiativen RF-Test nach dem jetzigen Stand der Technik ermittelt wurden:

| Serum-Nr. | **ELISA RF/IgM** (IU/ml) | **Nephelometrische RF- Bestimmung** (IU/ml) |
|---|---|---|
| 1 | 236 | 228 |
| 2 | 65 | 84 |
| 3 | 101 | 104 |
| 4 | 42 | 52 |
| 5 | 62 | 60 |
| 6 | 16 | 21 |

## Patentansprüche

1. Immunchemisches Verfahren zum Nachweis oder zur Bestimmung von Rheumafaktoren in einer Probe mittels eines spezifischen Bindungspartners, dadurch gekennzeichnet, daß dieser ein Immunkomplex ist, welcher durch Mischen eines Antigens und eines dagegen gerichteten Antikörpers in wässriger Lösung hergestellt und anschließend an einer Festphase immobilisiert wird.

2. Verfahren nach Anspruch 1, dadurch gekennzeichnet, daß der Immunkomplex aus Antiseren von immunisierten Tieren mit einem Antigen humanen, tierischen oder pflanzlichen Ursprungs hergestellt wurde, das nicht der Analyt ist.

3. Verfahren nach Anspruch 1 und 2, dadurch gekennzeichnet, daß das Verhältnis im Immunkomplex von Antikörper : Antigen = 1: 0,05 bis 5 beträgt.

4. Verfahren nach Anspruch 1 bis 3, dadurch gekennzeichnet, daß das Antiserum vom Schaf ist.

5. Verfahren nach Anspruch 1 bis 3, dadurch gekennzeichnet, daß das Antiserum vom Kaninchen ist.

6. Verfahren nach Anspruch 1 bis 5, dadurch gekennzeichnet, daß die Auswertung nach einer partikelverstärkten immunologischen Methode erfolgt.

7. Verfahren zur Herstellung eines Reagenzes zur Verwendung in dem Verfahren nach Anspruch 1, dadurch gekennzeichnet, daß der Immunkomplex durch Mischen eines Antigens und eines dagegen gerichteten Antikörpers in wässriger Lösung hergestellt und anschließend an einer Festphase immobilisiert wird.

8. Verfahren nach Anspruch 7, dadurch gekennzeichnet, daß es sich um ein Latex-Reagenz handelt.

9. Verfahren gemäß Anspruch 7 oder 8, dadurch gekennzeichnet, daß der Immunkomplex in Gegenwart von bis zu 50 Volumen % eines zyklischen Amids hergestellt wird.

10. Reagenz zum Nachweis oder zur Bestimmung von Rheumafaktoren, erhältlich durch ein Verfahren gemäß einem der Ansprüche 7, 8 oder 9.

## Claims

1. An immunochemical method for the detection or determination of rheumatoid factors in a sample by means of a specific binding partner, wherein the latter is an immune complex which is prepared by mixing an antigen and an antibody directed against it in aqueous solution and is subsequently immobilized on a solid phase.

2. The method as claimed in claim 1, wherein the immune complex has been prepared from antisera from animals immunized with an antigen of human, animal or vegetable origin that is not the analyte.

3. The method as claimed in claims 1 and 2, wherein the antibody:antigen ratio in the immune complex is 1:0.05 to 5.

4. The method as claimed in claims 1 to 3, wherein the antiserum is from sheep.

5. The method as claimed in claims 1 to 3, wherein the antiserum is from rabbit.

6. The method as claimed in claims 1 to 5, wherein the evaluation takes place using a particle-boosted immunological method.

7. A process for the preparation of a reagent for use in the method as claimed in claim 1, wherein the immune complex is prepared by mixing an antigen and an antibody directed against it in aqueous solution and is subsequently immobilized on a solid phase.

8. The process as claimed in claim 7, wherein the reagent is a latex reagent.

9. The process as claimed in claim 7 or 8, wherein the immune complex is prepared in the presence of up to 50% by volume of a cyclic amide.

10. A reagent for the detection or determination of rheumatoid factors, obtainable by a process as claimed in any one of claims 7, 8 or 9.

## Revendications

1. Procédé immunochimique pour la détection ou la détermination de facteurs rhumatoïdes dans un échantillon au moyen d'un partenaire de liaison spécifique, caractérisé en ce que ce dernier est un complexe immun qui est produit en solution aqueuse, par mélange d'un antigène et d'un anticorps dirigé contre celui-ci, et est ensuite immobilisé sur une phase solide.

2. Procédé selon la revendication 1, caractérisé en ce que le complexe immun a été produit à partir d'anti-sérums d'animaux immunisés, avec un antigène d'origine humaine, animale ou végétale, qui n'est pas l'analyte.

3. Procédé selon les revendications 1 et 2, caractérisé en ce que le rapport, dans le complexe immun, de l'anticorps à l'antigène va de 1:0,05 à 1:5.

4. Procédé selon les revendications 1 à 3, caractérisé en ce que l'antisérum est un antisérum de mouton.

5. Procédé selon les revendications 1 à 3, caractérisé en ce que l'antisérum est un antisérum de lapin.

6. Procédé selon les revendications 1 à 5, caractérisé en ce que l'évaluation est effectuée selon une méthode immunologique à renforcement par des particules.

7. Procédé pour la préparation d'un réactif pour utilisation dans le procédé selon la revendication 1, caractérisé en ce que le complexe immun est produit en solution aqueuse, par mélange d'un antigène et d'un anticorps dirigé contre celui-ci, et est ensuite immobilisé sur une phase solide.

8. Procédé selon la revendication 7, caractérisé en ce qu'il s'agit d'un réactif à base de latex.

9. Procédé selon la revendication 7 ou 8, caractérisé en ce que le complexe immun est produit en présence de jusqu'à 50 % en volume d'un amide cyclique.

10. Réactif pour la détection ou pour la détermination de facteurs rhumatoïdes, pouvant être obtenu par un procédé selon l'une des revendications 7, 8 et 9.
